(19) Europäisches Patentamt
European Patent Office
Office européen des brevets

(11) Numéro de publication : **0 160 577 B1**

(12) # FASCICULE DE BREVET EUROPÉEN

(45) Date de publication du fascicule du brevet : **13.04.88**

(51) Int. Cl.⁴ : **C 07 C 53/21**, C 07 C 51/25

(21) Numéro de dépôt : **85400170.8**

(22) Date de dépôt : **01.02.85**

(54) **Procédé de préparation d'acides perfluoroalcane-carboxyliques.**

(30) Priorité : **14.02.84 FR 8402195**

(43) Date de publication de la demande : **06.11.85 Bulletin 85/45**

(45) Mention de la délivrance du brevet : **13.04.88 Bulletin 88/15**

(84) Etats contractants désignés : **AT BE CH DE FR GB IT LI NL SE**

(56) Documents cités :
DE-A- 2 120 364
FR-A- 2 103 459
GB-A- 577 481
GB-A- 774 103
US-A- 3 446 859

(73) Titulaire : **ATOCHEM**
**4 & 8, Cours Michelet La Défense 10**
**F-92800 Puteaux (FR)**

(72) Inventeur : **Blancou, Hubert**
**Le Sylvie Bâtiment A Rue Paul Rimbaud**
**F-34000 Montpellier (FR)**
Inventeur : **Commeyras, Auguste**
**6, Impasse des Ecoles**
**F-34960 Clapiers (FR)**
Inventeur : **Teissedre, Robert**
**Mas Veyrassy 305 rue des Quatre Vents**
**F-34100 Montpellier (FR)**

(74) Mandataire : **Leboulenger, Jean et al**
**ATOCHEM Département Propriété Industrielle**
**F-92091 Paris la Défense 10 Cédex 42 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 160 577 B1

Jouve, 18, rue St-Denis, 75001 Paris, France

**Description**

La présente invention concerne la fabrication des acides perfluoroalcane-carboxyliques $R_FCO_2H$, formule dans laquelle $R_F$ représente un radical perfluoroalkyle, linéaire ou ramifié, contenant de 1 à 20 atomes de carbone, de préférence 4 à 12.

Ces acides dont les sels sont utilisés comme agents tensioactifs dans la polymérisation du tétrafluoroéthylène ou comme émulseurs dans les mousses destinées à la lutte contre l'incendie, sont préparés industriellement par électrofluoration de chlorures d'acides alcane-sulfoniques ou par hydrolyse des perfluoro-iodo-alcanes $R_FI$ au moyen d'oléum. Cette dernière méthode entraîne cependant la perte de deux atomes de fluor par molécule de dérivé perfluoro-iodé.

Dans le brevet GB 577481 on a décrit la préparation d'acides fluoro-acétiques par oxydation au permanganate de composés fluoro-éthyléniques de formule :

$$CF_2X\text{-}CY = CYZ$$

dans laquelle Y et Z désignent l'hydrogène ou le chlore et X un atome d'hydrogène, de fluor ou de chlore, les deux symboles Y étant identiques. Comme exemples de tels composés fluoroéthyléniques sont mentionnés le trichloro-1,1,2 trifluoro-1,3,3 propène, le tétrachloro-1,1,2,3 difluoro-3,3 propène, le trifluoro-3,3,3 propène préparé par traitement du chloro-1 trifluoro-3,3,3 propène par KOH et le dichloro-1,2 difluoro-3,3 propène préparé par traitement du tétrachloropropène par $SbF_3$.

Selon le brevet FR 2 103 459, la réaction d'une iodure perfluoroalkylé avec une oléfine contenant au moins 2 atomes d'hydrogène sur la double liaison en présence d'une amine et d'un sel métallique fournit des produits d'addition, la réaction pouvant être schématisée comme suit :

$$R_FI + H_2C = CHX \longrightarrow R_F\text{-}CH_2\text{-}CHI\text{-}X$$

Ces produits d'addition iodés ne peuvent pas être transformés en acides perfluoro-carboxyliques par oxydation au permanganate.

Il a maintenant été trouvé un procédé avantageux pour la fabrication de ces acides perfluoroalcane-carboxyliques. Le procédé selon l'invention consiste à faire réagir un perfluoro-iodo-alcane $R_FI$ avec le trichloroéthylène dans un solvant acide en présence de zinc, puis à oxyder à l'aide de permanganate de potassium ou de sodium le perfluoroalkyl-2 dichloro-1,1 éthylène ainsi obtenu de formule $R_FCH = CCl_2$.

Dans ce procédé qui peut être schématiquement représenté comme suit :

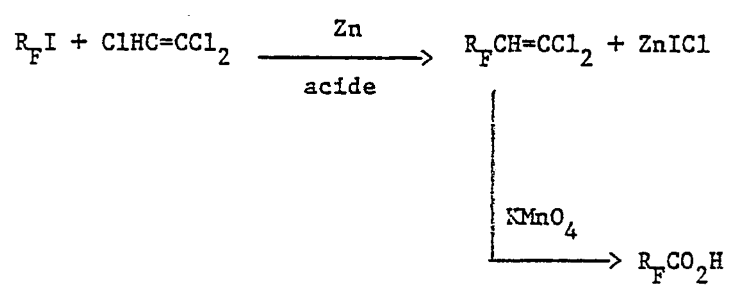


le perfluoro-iodo-alcane et le trichloroéthylène peuvent être engagés en quantité stœchiométrique. Cependant, pour limiter la formation de produits secondaires (essentiellement le perfluoro-hydroalcane $R_FH$ et le perfluoro-alcane $R_F\text{-}R_F$), il s'est révélé avantageux d'utiliser le trichloroéthylène en une quantité environ deux fois molaire par rapport au perfluoro-iodo-alcane.

La réaction du perfluoro-iodo-alcane et du trichloroéthylène est réalisée dans un solvant acide qui est de préférence l'acide propionique, mais peut être aussi un autre acide organique (par exemple l'acide formique ou acétique) ou un acide minéral dilué (par exemple l'acide chlorhydrique à 10 %) ou un mélange d'un acide organique ou minéral et d'un solvant organique tel que, par exemple, le benzène, le toluène ou le dioxanne. La quantité de solvant acide à utiliser peut varier dans de larges limites selon la nature du perfluoro-iodo-alcane engagé et de l'activation du zinc ; elle est généralement comprise entre environ 100 et 1 000 ml par mole de perfluoro-iodo-alcane.

Le zinc utilisé dans ce procédé est de la poudre de zinc commerciale, mais il peut être intéressant d'activer cette poudre selon les méthodes bien connues de l'homme du métier (Houben-Weyl 1973 XIII 2a, p. 570 à 574 et 815), par exemple par attaque avec un acide minéral fort comme l'acide chlorhydrique ou l'acide sulfurique ou par formation d'alliages avec d'autres métaux comme le sodium, le plomb, le mercure ou le cuivre. Le procédé selon l'invention nécessite une quantité stœchiométrique de zinc, mais on peut avantageusement utiliser un excès de zinc (5 à 50 %, en pratique 10 % environ) par rapport au perfluoro-iodo-alcane.

La réaction du perfluoro-iodo-alcane et du trichloroéthylène est habituellement effectuée à la pression et température ambiantes. Cependant, si on le désire, on peut opérer à une température plus élevée pouvant aller jusqu'à l'ébullition du mélange réactionnel.

Cette réaction peut être effectuée sous agitation. On a cependant constaté que la proportion de produits secondaires formés est plus faible lorsque le mélange n'est pas soumis à agitation et qu'on opère dans l'acide propionique avec un rapport molaire trichloroéthylène/$R_FI$ égal à 2, la réaction se produisant alors par diffusion lente de l'acide propionique dans le mélange $R_FI/CHCl = CCl_2$.

Les produits obtenus peuvent être isolés du mélange réactionnel par des moyens appropriés classiques tels que : lavage à l'eau, décantation, extraction, distillation ou filtration.

Le trifluorométhyl-2 dichloro-1,1 éthylène et son utilisation comme anesthésique sont déjà connus (voir, par exemple, J. Am. Chem. Soc. 64, 1157-9 ; J. Mol. Spectroscopie 7 ; 385 ; Anesthesiology 14, 466). Un composé de formule brute $C_4HCl_2F_5$, obtenu par distillation du produit brut de fluoruration de polychlorobutanes, est décrit dans l'ouvrage « Preparation, properties and technology of fluorine and organic fluoro compounds », Mc Graw-Hill 1951, 770-5). Les perfluoroalkyl-2 dichloro-1,1 éthylènes dans lesquels le radical perfluoroalkyle contient de 4 à 12 atomes de carbone sont nouveaux et, en tant que tels, font partie de la présente invention.

L'oxydation des perfluoroalkyl-2 dichloro-1,1 éthylènes en acides perfluoroalcane-carboxyliques est effectuée en milieu aqueux basique, l'agent alcalin le plus commode étant le carbonate de sodium, et à une température d'au moins 80 °C pouvant aller jusqu'à la température d'ébullition du mélange réactionnel (environ 100 °C).

Les exemples suivants illustrent l'invention sans la limiter. Les pourcentages indiqués sont exprimés en poids, à l'exception de ceux relatifs aux analyses RMN qui sont des pourcentages molaires.

## Exemple I

6,4 g (0,11 mole) de zinc en poudre sont dispersés sous agitation dans un mélange de 26 g (0,2 mole) de trichloroéthylène et de 30 ml d'acide propionique. Il est alors ajouté en 2 heures à cette dispersion 44,6 g de $C_6F_{13}I$ (0,1 mole). La réaction est exothermique.

En fin de réaction, le mélange est traité par de l'eau et après décantation, la phase organique est successivement lavée avec une solution d'acide chlorhydrique à 20 %, puis avec une solution de carbonate de sodium à 20 % et enfin à l'eau. Après distillation sous vide, on obtient 29 g de perfluorohexyl-2 dichloro-1,1 éthylène Teb. $_{20mm}$ : 105 °C (rendement 69 %).

L'analyse RMN$^{19}$F du mélange réactionnel montrait la présence respective de 80 % de perfluorohexyl-2 dichloro-1,1 éthylène, 15 % de perfluorohydrohexane ($C_6F_{13}H$) et 5 % de perfluorododécane ($C_{12}F_{26}$).

Le perfluorohexyl-2 dichloro-1,1 éthylène a été identifié par analyse spectrale RMN$^{19}$F, RMN$^1$H, IR, masse.

## Exemple II

En opérant comme dans l'exemple I, mais avec seulement 13,2 g (0,1 mole) de trichloroéthylène, l'analyse RMN$^{19}$F du mélange réactionnel montrait la présence respective de 35 % de $C_6F_{13}CH = CCl_2$, 42 % de $C_6F_{13}H$ et 23 % de $C_{12}F_{26}$.

## Exemple III

En opérant comme dans l'exemple I, mais avec 39,6 g (0,3 mole) de trichloroéthylène, l'analyse RMN$^{19}$F du mélange réactionnel montrait la présence respective de 65 % de $C_6F_{13}CH = CCl_2$, 15 % de $C_6F_{13}H$ et 20 % de $C_{12}F_{26}$.

## Exemple IV

Le zinc, le trichloroéthylène, le perfluoro-iodo-hexane, puis l'acide propionique sont ajoutés successivement dans les mêmes proportions que dans l'exemple I dans un récipient cylindrique de faible section (diamètre : 40 mm). Le mélange est laissé sans agitation pendant 24 heures. Il est ensuite traité comme dans l'exemple I successivement par $H_2O$, HC1, $Na_2CO_3$, $H_2O$. La phase organique est ensuite distillée et l'on recueille 37 g de perfluorohexyl-2 dichloro-1,1 éthylène. Rendement : 89 %.

L'analyse RMN$^{19}$F du milieu réactionnel montrait la présence respective de perfluorohexyl-2 dichloro-1,1 éthylène (90 %) et de perfluorohydrohexane (10 %).

## Exemples V à VII

Le tableau suivant résume les résultats obtenus en opérant comme dans l'exemple I, mais en remplaçant l'acide propionique par une solution aqueuse d'acide chlorhydrique à 10 % (exemple V), par l'acide acétique pur (exemple VI) et par l'acide formique (exemple VII).

| | Exemple V | Exemple VI | Exemple VII |
|---|---|---|---|
| $C_6F_{13}CH=CCl_2$ | 30 % | 77 % | 59 % |
| $C_6F_{13}H$ | 35 % | 23 % | 5 % |
| $C_{12}F_{26}$ | 33 % | - | 21 % |
| $C_6F_{13}I$ | 2 % | - | 5 % |
| Produits non identifiés | - | - | 10 % |

Exemple VIII

On opère comme dans l'exemple I, mais en remplaçant $C_6F_{13}I$ par 34,6 g (0,1 mole) de $C_4F_9I$. On obtient ainsi $C_4F_9CH = CCl_2$ (85 %), $C_4F_9H$ (5 %) et divers produits non identifiés (10 %).

Exemple IX

20,8 g (0,05 mole) de $C_6F_{13}CH = CCl_2$ sont ajoutés à 15,8 g (0,1 mole) de $KMnO_4$ et 12 g de $CO_3Na_2.2H_2O$ dans 30 ml d'eau. Le mélange réactionnel est porté pendant 3 heures à 80 °C, puis filtré et le filtrat est traité avec 5 ml d'acide chlorhydrique concentré (400 g/1). L'acide perfluorohexane-carboxylique décante et est distillé. On obtient ainsi 16 g d'acide $C_6F_{13}CO_2H$ identifié par comparaison avec un échantillon authentique. Rendement : 88 %.

Exemple X

En opérant comme à l'exemple IX à partir du perfluorobutyl-2 dichloro-1,1 éthylène de l'exemple VIII, on obtient de la même façon l'acide $C_4F_9CO_2H$.

**Revendications**

1. Procédé de préparation d'acides perfluoroalcane-carboxyliques $R_FCO_2H$ où $R_F$ représente un radical perfluoroalkyle, linéaire ou ramifié, contenant de 1 à 20 atomes de carbone, caractérisé en ce que l'on fait réagir un perfluoro-iodo-alcane $R_FI$ avec le trichloroéthylène dans un solvant acide en présence de zinc, puis on oxyde le perfluoroalkyl-2 dichloro-1,1 éthylène $R_FCH = CCl_2$ ainsi obtenu, cette oxydation étant effectuée en milieu basique à l'aide de permanganate de potassium ou de sodium à une température d'au moins 80 °C.

2. Procédé selon la revendication 1, dans lequel le rapport molaire trichloroéthylène/perfluoro-iodo-alcane est égal à 2 environ.

3. Procédé selon la revendication 1 ou 2, dans lequel le solvant acide est l'acide formique, acétique ou propionique ou l'acide chlorhydrique dilué.

4. Procédé selon l'une des revendications 1 à 3, dans lequel la réaction du perfluoro-iodo-alcane et du trichloroéthylène est effectuée sans agitation.

5. Procédé selon la revendication 4, dans lequel le solvant acide est l'acide propionique.

6. Procédé selon l'une des revendications 1 à 5, dans lequel l'oxydation est effectuée en présence de carbonate de sodium.

7. Procédé selon l'une des revendications 1 à 6, dans lequel on utilise un perfluoro-iodo-alcane contenant de 4 à 12 atomes de carbone.

8. Les perfluoroalkyl-2 dichloro-1,1 éthylènes : $R_FCH = CCl_2$, dans lesquels $R_F$ représente un radical perfluoroalkyle, linéaire ou ramifié, contenant de 4 à 12 atomes de carbone.

**Claims**

1. Process for preparing perfluoroalkane-carboxylic acids $R_FCO_2H$ wherein $R_F$ denotes a straight-chain or branched perfluoroalkyl radical containing from 1 to 20 carbon atoms, characterized in that a perfluoroiodo-alkane $R_FI$ is reacted with trichloroethylene in an acidic solvent in the presence of zinc, and then the resulting 2-perfluoroalkyl-1,1-dichloroethylene $R_FCH = CCl_2$. is oxidised in a basic medium with the aid of potassium or sodium permanganate at a temperature of at least 80 °C.

2. Process according to Claim 1, in which the molar ratio trichloroethylene/perfluoroiodoalkane is approximately 2.

3. Process according to Claim 1 or 2, in which the acidic solvent is formic, acetic or propionic acid or dilute hydrochloric acid.

4. Process according to any of Claims 1 to 3, in which the reaction of the perfluoroiodoalkane with trichloroethylene is carried out without stirring.

5. Process according to Claim 4, in which the acidic solvent is propionic acid.

6. Process according to any one of Claims 1 to 5, in which the oxidation is carried out in the presence of sodium carbonate.

7. Process according to any one of Claims 1 to 6, in which a perfluoroiodoalkane containing from 4 to 12 carbon atoms is employed.

8. The 2-perfluoroalkyl-1,1-dichloro-ethylenes : $R_FCH = CCl_2$, wherein $R_F$ denotes a straight-chain or branched perfluoroalkyl radical containing from 4 to 12 carbon atoms.

**Patentansprüche**

1. Verfahren zur Herstellung von Perfluoralkancarbonsäuren der Formel $R_FCO_2H$, in der $R_F$ einen linearen oder verzweigten Perfluoralkylrest mit 1 bis 20 Kohlenstoffatomen darstellt, dadurch gekennzeichnet, daß man ein Perfluoralkyljodid $R_FJ$ mit Trichlorethylen in einem sauren Lösungsmittel in Gegenwart von Zink reagieren läßt und anschließend das so erhaltene 2-Perfluoralkyl-1,1-Dichlorethylen $R_FCH = CCl_2$ oxidiert, wobei diese Oxidation in basischem Milieu mit Hilfe von Kalium oder Natriumpermanganat bei einer Temperatur von wenigstens 80 °C durchgeführt wird.

2. Verfahren nach Anspruch 1, bei dem das molare Verhältnis Trichlorethylen/Perfluoralkyljodid bei ungefähr 2 liegt.

3. Verfahren nach Anspruch 1 oder 2, bei dem das saure Lösungsmittel Ameisensäure, Essigsäure, Propionsäure oder verdünnte Chlorwasserstoffsäure ist.

4. Verfahren nach einem der Ansprüche 1 bis 3, bei dem die Reaktion des Perfluoralkyljodids und des Trichlorethylens ohne Rührung durchgeführt wird.

5. Verfahren nach Anspruch 4, bei dem das saure Lösungsmittel Propionsäure ist.

6. Verfahren nach einem der Ansprüche 1 bis 5, bei dem die Oxidation in Gegenwart von Natriumcarbonat durchgeführt wird.

7. Verfahren nach einem der Ansprüche 1 bis 6, bei dem man ein Perfluoralkyljodid mit 4 bis 12 Kohlentstoffatomen verwendet.

8. 2-Perfluoralkyl-1,1-dichlorethylene der Formel $R_FCH = CCl_2$, in denen $R_F$ einen linearen oder verzweigten Perfluoralkylrest mit 4 bis 12 Kohlenstoffatomen darstellt.